# EUROPÄISCHE PATENTSCHRIFT

(11) **EP 3 378 510 B1**
(45) Veröffentlichungstag und Bekanntmachung des Hinweises auf die Patenterteilung: **11.01.2023**
(21) Anmeldenummer: 18157539.0
(22) Anmeldetag: 20.02.2018
(51) Int. Cl.: A61M 5/20

(54) **FLASCHENHALTER FÜR EINE SPRITZE**
BOTTLE HOLDER FOR A SYRINGE
PORTE-BOUTEILLES POUR UN PISTOLET

(30) Priorität: 22.03.2017 DE 102017106149
(43) Veröffentlichungstag der Anmeldung: 26.09.2018
(73) Patentinhaber: Henke-Sass, Wolf GmbH, 78532 Tuttlingen (DE)
(72) Erfinder: EISELE, Melanie, 78573 Wurmlingen (DE); GAMBONI, Fabian, 6415 Arth (CH)
(74) Vertreter: Patentanwälte Geyer, Fehners & Partner mbB

(56) Entgegenhaltungen:
- DE-A1-102011 081 797
- DE-U1- 9 409 867
- DE-U1-202010 000 281
- US-A1- 2012 046 635

## Beschreibung

Die vorliegende Erfindung betrifft einen Flaschenhalter für eine Spritze (insbesondere für eine veterinärmedizinische Spritze), wobei der Flaschenhalter eine Anschlagfläche und einen über die Anschlagfläche vorstehenden und sich in eine erste Richtung erstreckenden Einstichhohldorn, eine Führung sowie ein erstes und ein zweites Klemmelement aufweist.

Da solche Spritzen häufig mit Flaschen unterschiedlicher Größe verwendet werden sollen, tritt die Schwierigkeit auf, die Flaschen mittels des Flaschenhalters sicher zu haltern, da Flaschen unterschiedlicher Größe unterschiedliche Abmessungen aufweisen. So kann z.B. der Durchmesser einer auf dem Flaschenhals angeordneten Verschlusskappe und die Höhe der Verschlusskappe unterschiedlich sein. Daher wird bisher für jede Größe der aufzunehmenden Flasche ein individueller Flaschenhalter oder ein Flaschenhalter, bei dem zumindest ein Teil, wie z.B. ein Adapterstück, auszutauschen ist, vorgesehen. Dies führt zu erhöhtem Aufwand bei einem Wechsel von einer Flasche einer ersten Größe zu einer Flasche einer zweiten Größe.

Die DE 20 2010 000 281 U1 sowie die DE 10 2011 081 797 A1 zeigen jeweils einen Flaschenhalter der eingangs genannten Art. Die DE 94 09 867 U1 zeigt eine Universalaufhängung, die insbesondere für Infusionsflaschen geeignet ist und mehrere an zwei Rahmenschenkeln drehbar gelagerte Ringe zum Halten von Infusionsflaschen aufweist. Die US 2012/0046635 A1 zeige eine Nadelschutzvorrichtung, die zur Befestigung an einem Flüssigkeitsbehälter geeignet ist.

Ausgehend hiervon ist es daher Aufgabe der Erfindung, einen verbesserten Flaschenhalter der eingangs genannten Art bereitzustellen.

Die Erfindung ist im Anspruch 1 definiert. Vorteilhafte Ausgestaltungen sind in den abhängigen Ansprüchen angegeben.

Da der erfindungsgemäße Flaschenhalter das erste und zweite Klemmelement aufweist, können zumindest zwei unterschiedliche Höhen von Kappen von aufzunehmenden Flaschen sicher gehalten und arretiert werden. Bevorzugt steht zumindest eines der Klemmelemente in eine Hinterschneidung hinter der Kappe im Bereich des Halses der Flasche ein.

Der erfindungsgemäße Flaschenhalter für eine Spritze weist eine Anschlagfläche, einen über die Anschlagfläche vorstehenden und sich in eine erste Richtung erstreckenden Einstichhohldorn, eine Führung und ein erstes und ein zweites Klemmelement auf, die entlang der ersten Richtung aufeinander angeordnet sind und in der Führung quer zur ersten Richtung entlang einer zweiten Richtung bewegbar gelagert sind. Jedes der Klemmelemente umfasst eine Durchgangsöffnung und ist zwischen einer Einführposition und einer Klemmposition hin und her bewegbar, wobei, wenn die Klemmelemente in der Einführposition stehen, ein Hals der Flasche (einschließlich der Kappe) durch die Durchgangsöffnung bewegt werden kann, bis das vordere Ende der Flasche an der Anschlagfläche anliegt, so dass der Einstichhohldorn eine Schicht an dem vorderen Ende der Flasche durchsticht, und wobei mindestens eines der Klemmelemente, wenn es in seiner Klemmposition steht, gegen den Hals der Flasche, die mit ihrem vorderen Ende an der Anschlagfläche anliegt, drückt, um dadurch die Flasche im Halter einzuklemmen.

Wie bereits dargelegt wurde, steht dabei bevorzugt zumindest eines der Klemmelemente in einer Hinterschneidung hinter der Kappe am Hals der Flasche ein.

Jedes Klemmelement kann mindestens einen Federarm aufweisen, der das in der Führung gelagerte Klemmenelement mit einer Kraft in Richtung zur Klemmposition hin beaufschlagt. Insbesondere kann jedes Klemmelement zwei Federarme aufweisen, die bevorzugt V-förmig angeordnet sind.

Bevorzugt ist jedes Klemmelement einstückig ausgebildet. Beispielsweise kann das Klemmelement aus Kunststoff hergestellt sein. Insbesondere kann das Klemmelement als Spritzgussteil gebildet sein.

Ferner kann bei dem mindestens einem Federarm jedes Klemmelementes ein erster und ein zweiter Anschlagsteg, die sich von der Durchgangsöffnung weg erstrecken, vorgesehen sein, wobei die beiden Anschlagstege Endpositionen der Bewegung des Klemmelementes in der zweiten Richtung festlegen. Dazu kann die Führung entsprechende Anschlagflächen aufweisen.

Der erfindungsgemäße Flaschenhalter kann ein hüllenförmiges oder korbförmiges Halteteil mit einer Wandung aufweisen, wobei die Führung zwei gegenüberliegende Führungsöffnungen in der Wandung umfasst. Die beiden Öffnungen können durch zwei Stege voneinander getrennt sein. Die Stege können Anschlagflächen für die Anschlagstege bereitstellen.

Der Rand der Durchgangsöffnung eines der Klemmelemente kann (in der ersten Richtung auf das Klemmelement gesehen) zwei entlang der zweiten Richtung gegenüberliegende abgerundete Enden unterschiedlicher Größe umfassen, die mit zwei Verbindungsabschnitten (beispielsweise geradlinige Verbindungsabschnitte) verbunden sind. Insbesondere kann die Form der Durchgangsöffnung als oval, eiförmig bzw. tropfenförmig beschrieben werden.

Die abgerundeten Enden können beliebige Krümmungsverläufe oder z. B. Kreisbahnabschnitte aufweisen. Es ist jedoch auch ein Abschnitt einer Ellipsenbahn möglich. Auch kann die abgerundete Form durch z. B. lineare Abschnitte angenähert sein.

Bevorzugt ist das Ende mit der größeren Abrundung so ausgelegt, dass es größer ist als der maximale Außendurchmesser einer Kappe von mehreren aufzunehmenden Medikamentenflaschen, die sich in ihrer Größe unterscheiden. Die Größe des kleineren abgerundeten Endes ist bevorzugt so gewählt, dass sie kleiner ist als der Außendurchmesser des Halses der Flasche, die den Hals mit geringstem Außendurchmesser aufweist.

In dieser Art und Weise kann bevorzugt gewährleistet werden, dass das in die Hinterschneidung einstehende Klemmelement bei eingesetzter Flasche jeweils mit beiden Verbindungsabschnitten aufliegt. Damit kann eine sichere Fixierung erreicht werden.

Der erfindungsgemäße Flaschenhalter kann für einen Satz von Flaschen unterschiedlicher Größe ausgelegt sein. Dadurch wird ein System aus Flaschenhalter (gegebenenfalls zusammen mit der Spritze) und aus dem Satz von Flaschen bereitgestellt.

Die Durchgangsöffnung der Klemmelemente kann z. B. für Außendurchmesser der Kappe von 12 bis 35 mm ausgelegt sein. Ferner können die Klemmelemente für Kappenhöhen von 5 bis 25 mm und insbesondere von 5 bis 17 mm oder 7 bis 14 mm ausgelegt sein.

Die Durchgangsöffnung kann in mindestens einem der Klemmelemente vollständig umrandet sein. Somit umfasst das Klemmelement einen ringförmigen Klemmabschnitt, in dem die Durchgangsöffnung ausgebildet ist.

Mindestens eines der Klemmelemente kann so ausgebildet sein, dass es um eine Achse entlang der zweiten Richtung um 180° drehsymmetrisch ist. Damit wird in vorteilhafter Weise erreicht, dass eine fehlerhafte Anordnung des Klemmelementes in der Führung verhindert wird.

Mindestens eines der Klemmelemente kann entlang der ersten Richtung eine konstante Dicke aufweisen. Ferner können die Klemmelemente unmittelbar aufeinander liegen. Die Klemmelemente können alle gleich ausgebildet sein.

Es ist jedoch auch möglich, dass Klemmelemente unterschiedlicher Dicken aufeinander positioniert werden.

Es wird ferner ein Flaschenhalter für eine Spritze bereitgestellt, wobei der Flaschenhalter eine Anschlagfläche, einen über die Anschlagfläche vorstehenden und sich in eine erste Richtung erstreckenden Einstichhohldorn, eine Führung und mindestens ein Klemmelement aufweist, das in der Führung quer zur ersten Richtung entlang einer zweiten Richtung bewegbar gelagert ist, wobei das zumindest eine Klemmelement eine Durchgangsöffnung umfasst und zwischen einer Einführposition und einer Klemmposition hin und her bewegbar ist. Der Rand der Durchgangsöffnung des mindestens einen Klemmelementes kann (in der ersten Richtung auf das Klemmelement gesehen) zwei entlang der zweiten Richtung gegenüberliegende abgerundete Enden unterschiedlicher Größe umfassen, die mit zwei Verbindungsabschnitten (bevorzugt geradlinigen Verbindungsabschnitten) verbunden sind. Wenn das zumindest eine Klemmelement in der Einführposition steht, kann ein Hals der Flasche durch die Durchgangsöffnung bewegt werden, bis das vordere Ende der Flasche an der Anschlagfläche anliegt, so dass der Einstichhohldorn eine Schicht am vorderen Ende der Flasche durchsticht. Wenn das mindestens eine Klemmelement in seiner Klemmposition steht, kann es gegen den Hals der Flasche, die mit ihrem vorderen Ende an der Anschlagfläche anliegt, drücken, um damit die Flasche im Halter einzuklemmen. Insbesondere kann das mindestens eine Klemmelement in seiner Klemmposition an einer Hinterschneidung am Hals der Flasche anliegen und somit bevorzugt eine Kraft in Richtung zur Anschlagfläche hin auf die Flasche ausüben. Dieser Flaschenhalter kann in gleicher Weise wie der bereits beschriebene Flaschenhalter weitergebildet werden.

Ferner wird eine Spritze mit einem erfindungsgemäßen Flaschenhalter (einschließlich der beschriebenen Weiterbildungen) bereitgestellt. Bei der Spritze kann es sich um eine veterinärmedizinische Spritze handeln. Insbesondere kann die Spritze als Selbstfüllerspritze ausgebildet sein.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine perspektivische Ansicht einer ersten Ausführungsform der erfindungsgemäßen Spritze mit einem erfindungsgemäßen Halter;
- Fig. 2: eine perspektivische Ansicht einer am Halter 2 gemäß Fig. 1 aufzunehmenden Flasche 3;
- Fig. 3: eine Vorderansicht der Spritze 1 mit Halter 2 gemäß Fig. 1;
- Fig. 4: eine Schnittansicht entlang der Schnittlinie A-A von Fig. 3;
- Fig. 5: eine perspektivische Ansicht des ersten Klemmelementes 29;
- Fig. 6: eine Seitenansicht des ersten Klemmelementes 29 von Fig. 5;
- Fig. 7: eine Vorderansicht des ersten Klemmelementes 29 von Fig. 5;
- Fig. 8: eine perspektivische Ansicht des Halteteils 15;
- Fig. 9: eine Vorderansicht des Halteteils 15 von Fig. 8;
- Fig. 10: eine rechte Seitenansicht des Halteteils 15 von Fig. 8;
- Fig. 11: eine perspektivische Ansicht einer weiteren Flasche 3, und
- Fig. 12: eine Schnittansicht der erfindungsgemäßen Spritze mit dem erfindungsgemäßen Halter 2 in gleicher Weise wie in Fig. 4, wobei die Flasche 3 gemäß Fig. 11 im Halter 2 fixiert ist.

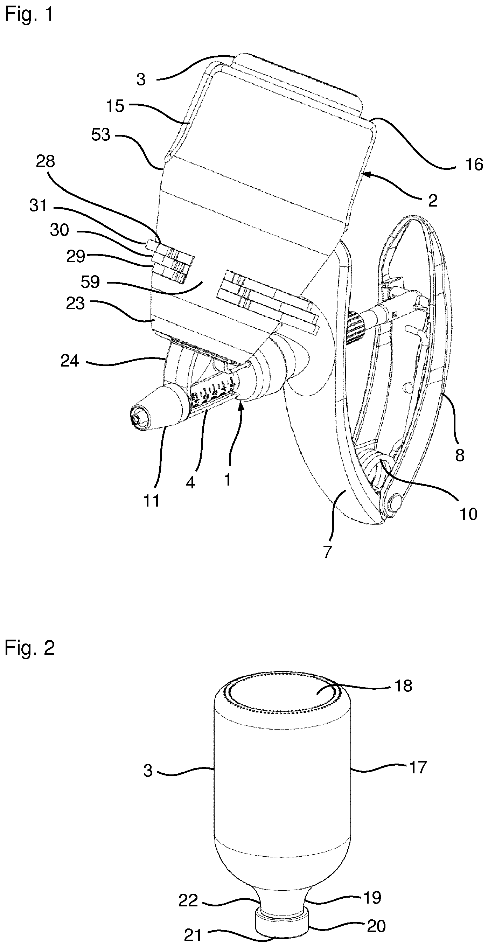

Bei der in Fig. 1 gezeigten Ausführungsform ist eine erfindungsgemäße Spritze 1 mit einem erfindungsgemäßen Flaschenhalter 2 für eine Flasche 3, die insbesondere eine Medikamentenflasche 3 sein kann, ausgerüstet.

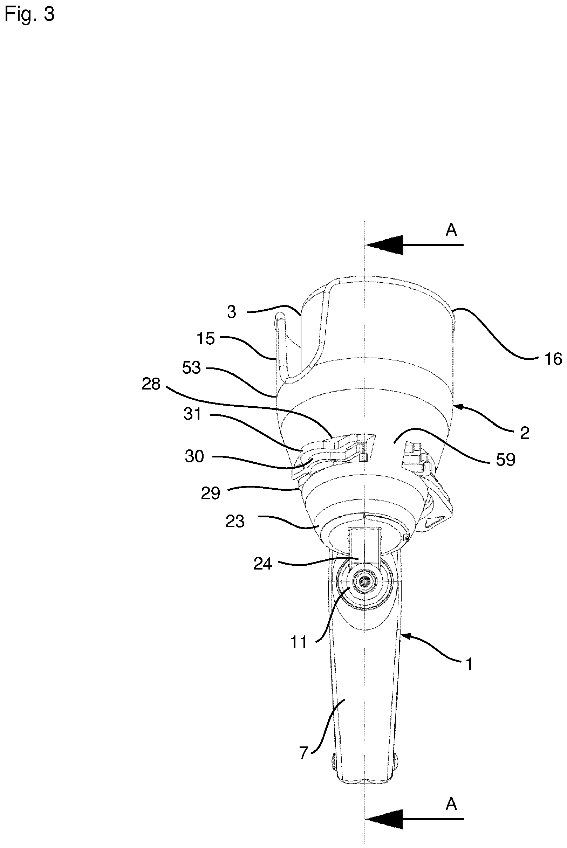

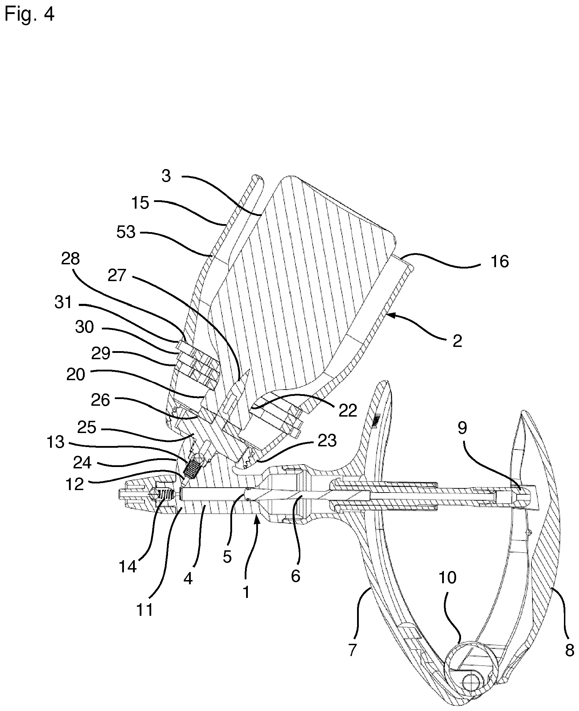

Die Spritze 1 kann z.B. als Selbstfüllerspritze für veterinärmedizinische Anwendungen ausgebildet sein und umfasst, wie insbesondere in den Fig. 1, 3 und 4 gezeigt ist, einen Spritzenzylinder 4, in den ein vorderes Ende 5 einer Kolbenstange 6 einsteht und in Längsrichtung des Spritzenzylinders 4 verschiebbar ist. Zur Betätigung der Kolbenstange 6 ist an einem Handgriff 7 schwenkbar ein Betätigungshebel 8 befestigt, der mit einem hinteren Ende 9 der Kolbenstange 6 verbunden ist. Zwischen dem Handgriff 7 und dem Bedienungshebel 8 ist eine Feder 10 angeordnet, die den Betätigungshebel 8 in der in Fig. 4 gezeigten Grundstellung hält.

Wenn der Betätigungshebel 8 von der in Fig. 4 gezeigten Grundstellung gegen die Federkraft zum Handgriff 7 hin verschwenkt wird, wird das vordere Ende 5 der Kolbenstange 6 im Spritzenzylinder 4 zum vorderen Ende 11 der Spritze 1 hin geschoben, so dass ein sich im Spritzenzylinder 4 befindendes Fluid oder eine sich im Spritzenzylinder 4 befindliche Flüssigkeit (bei dem Fluid oder der Flüssigkeit kann es sich um ein Medikament handeln) über das vordere Ende 11 abgegeben wird.

Wenn keine Kraft mehr auf den Betätigungshebel 8 ausgeübt wird, wird dieser aufgrund der Feder 10 in die in Fig. 4 gezeigte Grundstellung zurückgeschwenkt. Bei dieser Bewegung der Kolbenstange 6 von links nach rechts in Fig. 4 gesehen wird aus der Medikamentenflasche 3 das Medikament in den Spritzenzylinder 4 gefüllt. Dies wird dadurch erreicht, dass eine Fluidverbindung 12 zwischen der Medikamentenflasche 3 und dem Spritzenzylinder 4 vorliegt, wobei in der Fluidverbindung 12 ein erstes Rückschlagventil 13 angeordnet ist, das dafür sorgt, dass bei einer Bewegung des vorderen Endes 5 der Kolbenstange 6 von links nach rechts in Fig. 4 die Fluidverbindung 11 offen ist und somit das Medikament aus der Medikamentenflasche 3 in den Spritzenzylinder 4 gesaugt wird. Bei der entgegengesetzten Bewegung der Kolbenstange 6 ist das erste Rückschlagventil 13 geschlossen, so dass das Medikament über das vordere Ende 11 der Spritze 1 abgegeben werden kann. Dazu ist zwischen dem vorderen Ende 11 der Spritze 1 und dem Spritzenzylinder 4 ein zweites Rückschlagventil 14 angeordnet, das bei einer Bewegung des vorderen Endes 5 der Kolbenstange 6 von rechts nach links in Fig. 4 eine Fluidverbindung zwischen dem Spritzenzylinder 4 und dem vorderen Ende 11 öffnet und bei entgegengesetzter Bewegung diese Fluidverbindung schließt. An dem vorderen Ende 11 kann eine Injektionskanüle (nicht gezeigt) befestigt sein.

Wie insbesondere der Schnittdarstellung in Fig. 4 entnommen werden kann, umfasst der Flaschenhalter 2 ein im wesentlichen hohlzylinderförmig ausgebildetes Halteteil 15, das sich zum Spritzenzylinder 4 hin verjüngt und dessen vom Spritzenzylinder 4 abgewandtes Ende 16 offen ist. Das Halteteil 15 weist eine Wandung 53 auf und die Form des Halteteils 15 kann auch als korb-, hüllen- oder topfartig bezeichnet werden und ist im Wesentlichen an die Form der zu haltenden Medikamentenflaschen 3 angepasst.

Wie in Fig. 2 gezeigt ist, weist eine Medikamentenflasche 3 üblicherweise einen beispielsweise zylinderförmigen Körper 17 auf, der an einem Ende mit einem Boden 18 verschlossen ist und sich am entgegengesetzten Ende zu einem Hals 19 verjüngt, dessen offenes Ende mit einer Kappe 20 (z.B. eine Aluminium-Kappe) verschlossen ist, die eine durchstechbare Schicht 21 vor dem offenen Ende des Halses 19 aufweist. Wie in Fig. 2 gezeigt ist, ist der Außendurchmesser der Kappe 20 größer als der Außendurchmesser des Halses 19 in diesem Bereich, so dass eine Hinterschneidung 22 vorliegt. Die in Fig. 2 gezeigte Flasche 3 weist ein Volumen von 100 ml auf. Der Außendurchmesser der Kappe 20 beträgt 20 mm und die Höhe der Kappe 20 beträgt 7 mm.

Das Halteteil 15 ist, wie in Fig. 4 ersichtlich ist, mit seinem dem Ende 16 entgegengesetzten verjüngten Ende 23 an einem Aufnahmeabschnitt 24 des Spritzenzylinders 4 befestigt. In der vorliegenden Ausführungsform ist eine Schraubverbindung realisiert. Es ist jedoch jede andere Art von Verbindung, insbesondere jede andere Art von dauerhafter Verbindung, möglich. In dem Aufnahmeabschnitt 24 sitzt ein Anschlag 25 mit einer Anschlagfläche 26 und einem über die Anschlagfläche vorstehenden Einstichhohldorn 27.

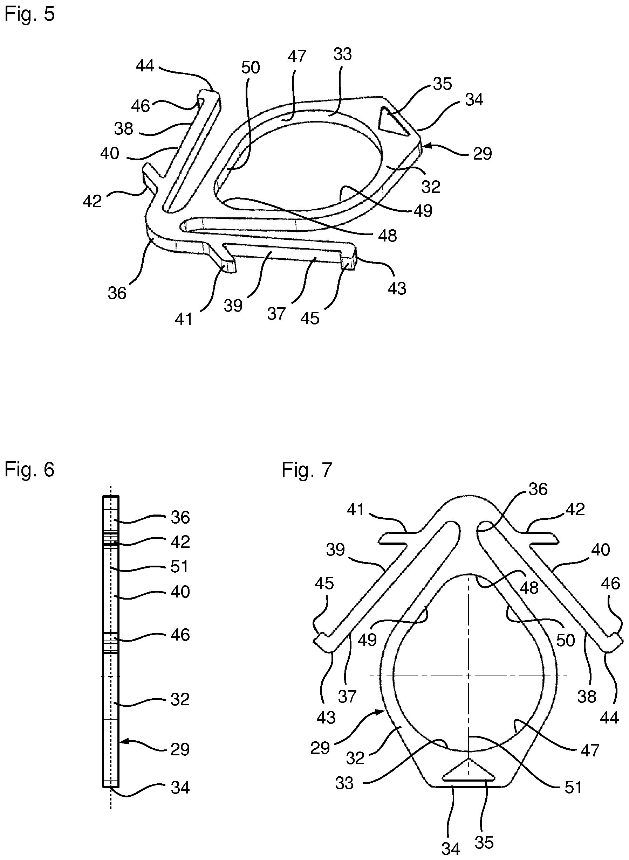

Ferner weist das Halteteil 15 eine Führung 28 auf, in der drei Klemmelemente 29, 30, 31 in einer Richtung quer zur ersten Richtung, in der sich der Einstichhohldorn 27 erstreckt, bewegbar gelagert sind. Die Klemmelemente 29 bis 31 (die auch als Schnapper 29-31 bezeichnet werden können) liegen in der ersten Richtung aufeinander und können in der Führung 28 voneinander unabhängig bewegt werden. Die drei Klemmelemente 29 bis 31 sind gleich ausgebildet, so dass nachfolgend der Aufbau des ersten Klemmelementes 29 im Detail unter Bezugnahme auf die Fig. 5 bis 7 beschrieben wird.

Das Klemmelement 29 weist einen Klemmabschnitt 32 mit einer Durchgangsöffnung 33 auf und kann daher auch als ringförmig bezeichnet werden. Der Klemmabschnitt 32 weist ein erstes Ende 34 mit einer dreieckförmigen Markierung 35 auf, die eine Schieberichtung für das Einführen der Medikamentenflasche 3 anzeigt, wie nachfolgend noch im Detail beschrieben wird. Die Markierung 35 kann jedoch auch weggelassen werden.

An dem dem ersten Ende 34 gegenüberliegenden zweiten Ende 36 des Klemmabschnitts 32 sind zwei Federarme 37, 38 in einer V-förmigen Anordnung ausgebildet, die jeweils einen geradlinig verlaufenden Abschnitt 39, 40 aufweisen, der vom Klemmabschnitt 32 beabstandet ist. Im Bereich des zweiten Endes 36 erstreckt sich von jedem Abschnitt 39, 40 jeweils ein erster Anschlagsteg 41, 42 in einer Richtung weg vom Klemmabschnitt 32. Des Weiteren weist jeder Federarm 37, 38 an seinem freien Ende 43, 44 einen zweiten Anschlagsteg 45, 46 auf, die sich jeweils weg vom Klemmabschnitt 32 erstrecken. Das erste Klemmelement 29 weist eine konstante Dicke auf, so dass man es unter diesem Aspekt auch als plattenförmig bezeichnen kann.

Wie insbesondere den Darstellungen in Fig. 5 und 7 zu entnehmen ist, ist die Durchgangsöffnung 33 nicht rein kreisförmig ausgebildet, sondern weist quasi eine Tropfenform, eine ovale Form oder Eiform auf, die sich dadurch ergibt, dass am ersten Ende 34 die Durchgangsöffnung 33 durch einen ersten Kreisbahnabschnitt 47 und am zweiten Ende durch einen zweiten Kreisbahnabschnitt 48 mit kleinerem Radius als der des ersten Kreisbahnabschnittes 47 begrenzt ist, wobei die beiden Kreisbahnabschnitte 47 und 48 durch zwei sich geradlinig erstreckende Verbindungsabschnitte 49, 50 verbunden sind. Der Abstand der beiden Verbindungsabschnitte 49, 50 und somit die lichte Breite nimmt in Richtung zum zweiten Ende 36 hin ab.

Das erste Klemmelement 29 ist symmetrisch zu einer Mittelebene, in der die Mittelachse 51 des ersten Klemmelements 29 verläuft und die senkrecht auf der Zeichenebene gemäß Fig. 7 steht. Man kann auch sagen, dass das erste Klemmelement 29 zur Mittelachse 51 um 180° drehsymmetrisch ist. Ferner ist das erste Klemmelement 29 symmetrisch zu einer Ebene, in der die Mittelachse 51 gemäß der Darstellung in Fig. 6 verläuft und die senkrecht zur Zeichenebene in Fig. 6 steht. Somit sieht das erste Klemmelement 29 gemäß Fig. 7 gleich aus, wenn es in der Darstellung in Fig. 7 um die Mittelachse 51 um 180° gedreht wird. Die Markierung 35 kann nur auf einer Seite oder auf beiden Seiten ausgebildet sein. Es kann auch auf beiden Seiten keine Markierung vorhanden sein.

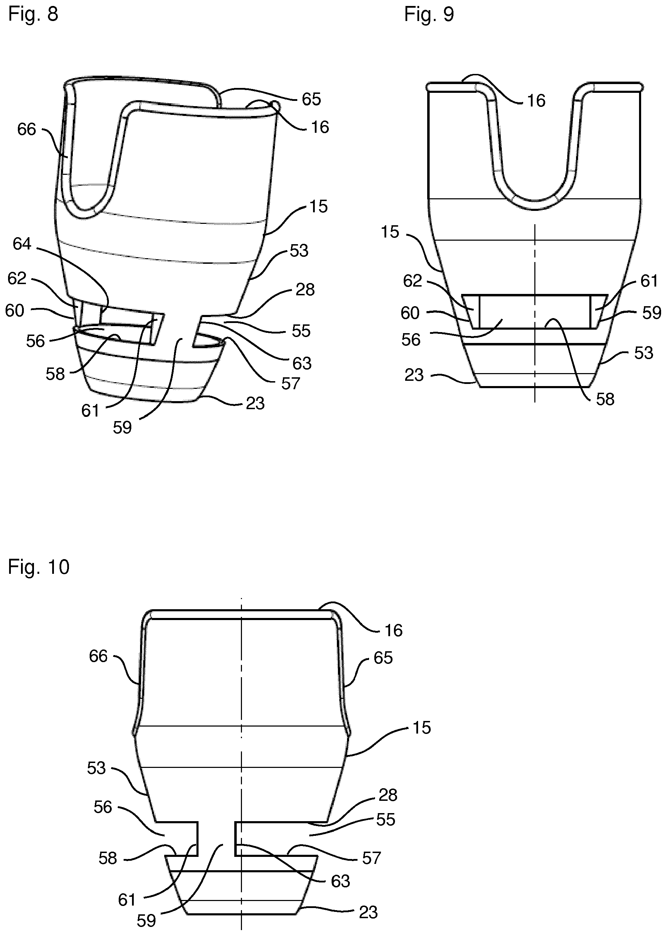

Die Führung 28 für die Klemmelemente 29-31 ist in dem Halteteil 15 durch eine erste und zweite Führungsöffnung 55, 56 in der Wandung 53 realisiert, wie in Fig. 8 bis 10 ersichtlich ist. Durch die beiden gegenüberliegenden Führungsöffnungen 55, 56 wird in der Wandung 53 eine erste und zweite Auflagefläche 57, 58 bereitgestellt. Die beiden Führungsöffnungen 55 und 56 sind durch einen ersten und zweiten Steg 59 und 60 getrennt, die jeweils eine erste Anschlagfläche 61 und 62 für den ersten Anschlagsteg 41 und 42 des ersten Klemmelements 29 und eine zweite Anschlagfläche 63, 64 für den zweiten Anschlagsteg 45, 46 des ersten Klemmelementes 29 bilden.

Wenn das erste Klemmelement 29 in der Führung 28 positioniert ist, liegt es auf den Auflageflächen 57 und 58 auf und drückt die Federarme 37, 38 mit ihren Abschnitten 39 und 40 von innen gegen die Stege 59 und 60, wobei die ersten Anschlagstege 41 und 42 der Federarme 37 und 38 in den Darstellungen von Fig. 8 und 10 links vom ersten bzw. zweiten Steg 59, 60 nach außen vorstehen und die zweiten Anschlagstege 45, 46 rechts vom ersten bzw. zweiten Steg 59,60 vorstehen. Da die Abmessungen des ersten Klemmelementes 29 und der Führung 28 so gewählt sind, dass die Stege 59 und 60 die Federarme 37, 38 nach innen zum Klemmabschnitt 32 hin drücken, führt dies bei dem Flaschenhalter 2 ohne eingesetzte Flasche 3 dazu, dass das erste Klemmelement 29 in Fig. 10 gesehen so weit nach rechts bewegt wird, bis der erste Anschlagsteg 41, 42 an der ersten Anschlagfläche 61 und 62 anliegt. Diese Position des ersten Klemmelementes 29 kann als Klemmposition bezeichnet werden.

Ferner ist es möglich, das erste Klemmelement 29 durch Drücken gegen sein erstes Ende 34 (und somit durch Beaufschlagen mit einer Kraft von rechts nach links in Fig. 10 gesehen) in Fig. 10 von rechts nach links so lange zu schieben, bis der zweite Anschlagsteg 45, 46 an der zweiten Anschlagfläche 63, 64 anliegt. Diese Position kann als Einführposition bezeichnet werden. Sobald keine Kraftbeaufschlagung auf das erste Klemmelement 29 gegen das erste Ende 34 mehr vorliegt, wird sich das erste Klemmelement 29 aufgrund der Rückstellkraft durch die Federarme 37, 38 wieder nach rechts bewegen (in Fig. 10 gesehen). Wenn keine Flasche eingeführt wird, endet die Bewegung sobald die ersten Anschlagstege 41 und 42 an den ersten Anschlagflächen 61 und 62 anliegen.

Wie bereits ausgeführt wurde, sind das zweite und dritte Klemmelement 30 und 31 gleich ausgebildet wie das erste Klemmelement 29. Bei der hier beschriebenen Ausführungsform liegt das zweite Klemmelement in der Führung 28 auf dem ersten Klemmelement 29 und liegt das dritte Klemmelement 31 in der Führung 28 auf dem zweiten Klemmelement 30. Die Höhe der beiden Führungsöffnungen 55 und 56 ist dabei so gewählt, dass die drei Klemmelemente 29 bis 31 sicher geführt sind.

Wenn eine Medikamentenflasche 3 in den Flaschenhalter 2 aufgenommen werden soll, sind die drei Klemmelemente 29 bis 31 in der beschriebenen Art und Weise durch Beaufschlagen mit einer Kraft auf ihr jeweiliges erstes Ende 34 in die Einführposition zu bringen, in der der Bereich mit dem ersten Kreisbahnabschnitt 47 der Durchgangsöffnung 33 möglichst konzentrisch zum Einstichhohldorn 27 liegt, so dass die Kappe 20 der Medikamentenflasche 3 durch die Durchgangsöffnungen 33 in Richtung zur Anschlagfläche 26 bewegt werden kann, bis die Kappe 20 an der Anschlagfläche 26 anliegt. Dabei wird die durchstechbare Schicht 21 mit dem Einstichhohldorn 27 durchstochen.

In dieser Stellung der Flasche 3 werden die Klemmelemente 29 bis 31 nicht mit einer Kraft beaufschlagt, so dass aufgrund der Rückstellkraft der Federarme 37 und 38 die drei Klemmelemente 29 bis 31 in Richtung ihrer Klemmposition hin zurückbewegt werden. Diese Bewegung endet jedoch, wie insbesondere in der Schnittansicht von Fig. 4 ersichtlich ist, an dem Flaschenhals 19, da die Klemmposition ohne eingesetzte Flasche für jedes der Klemmelemente 29 bis 31 weiter in Richtung zur ersten Führungsöffnung 55 hin liegt. Anders gesagt, liegen die ersten Anschlagstege 41 und 42 der Klemmelemente 29 bis 31 noch nicht an den ersten Anschlagflächen 61 und 62 an, so dass noch eine gewisse Federspannung vorliegt, die den die jeweilige Durchgangsöffnung 33 begrenzenden Rand des Klemmabschnitts 32 gegen den Flaschenhals 19 drückt. Wie in Fig. 4 ersichtlich ist, greifen die Klemmelemente 29 bis 31 gerade in die Hinterschneidung 22 ein, dies gilt insbesondere für das erste Klemmelement 29.

Aufgrund der beschriebenen tropfenförmigen Ausbildung der Durchgangsöffnungen 33 liegen Bereiche der geradlinigen Verbindungsabschnitte 49 und 50 auf der Kappe 20 von der Hinterschneidung 22 aus auf, so dass in vorteilhafter Weise auch eine Kraft in Richtung zur Anschlagfläche 26 hin auf die Flasche 3 ausgeübt wird, wodurch eine sichere Fixierung der Flasche 3 in dem Halter 2 gewährleistet ist. Natürlich liefert auch das zweite Klemmelement 30, das unmittelbar auf dem ersten Klemmelement 29 liegt, über das erste Klemmelement 29 eine Kraft in Richtung auf die Anschlagfläche 26. Dies gilt auch für das dritte Klemmelement 31, das über das zweite und erste Klemmelement 30 und 29 eine Kraftbeaufschlagung in Richtung auf die Einstichfläche 26 bewirkt.

Wenn die Medikamentenflasche 3 aus dem Halter entnommen werden soll (beispielsweise wenn sie leer ist), müssen lediglich die drei Klemmelemente 29 bis 31 durch Beaufschlagung mit einer Kraft auf ihr jeweiliges erstes Ende 34 in ihre Öffnungspositionen geschoben werden, so dass dann die Flasche 3 nach oben entnommen werden kann.

Durch die beschriebene Ausbildung des erfindungsgemäßen Flaschenhalters 2 wird nicht nur die beschriebene einfache und sichere Fixierung der Medikamentenflasche 3 erreicht. Es ist auch möglich, verschiedene Medikamentenflaschen 3, die sich durch ihre Größe und insbesondere die Größe des Durchmessers des Halses 19 und somit auch der Größe des Durchmessers der Kappe 20 und der Höhe der Kappe 20 und somit des Abstandes der Position der Hinterschneidung 22 von der Anschlagfläche 26, wenn die Flasche 3 im Halter 2 eingeführt ist, unterscheiden, sicher im Halter 2 aufzunehmen und zu fixieren.

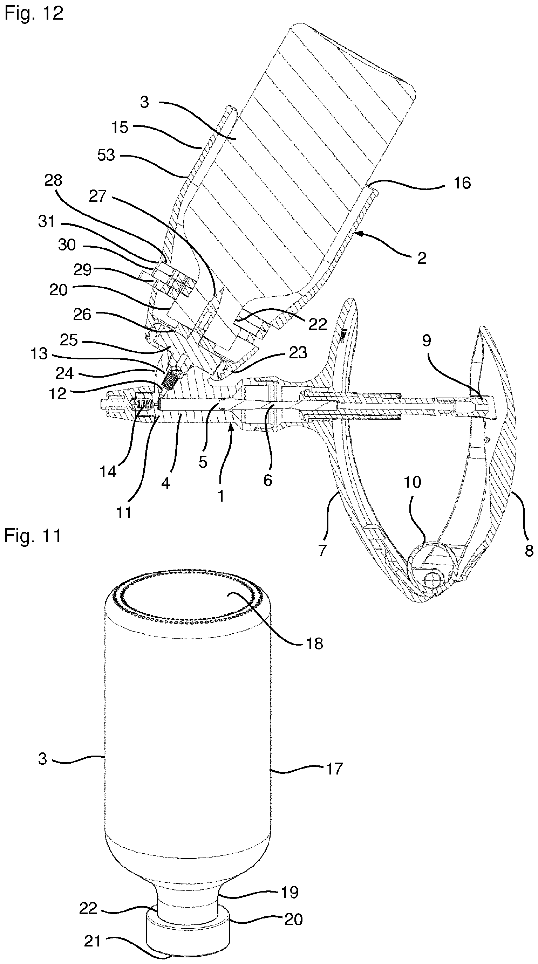

In Fig. 11 ist eine solche größere Flasche 3, die ein Volumen von 200 ml aufweist, gezeigt. Ein Vergleich mit Fig. 2 zeigt klar, dass die Höhe der Kappe 20 (hier 10,5 mm), der Außendurchmesser der Kappe (hier 30 mm) und der Halsdurchmesser größer sind. Die Flasche 3 aus Fig. 11 kann in der beschriebenen Art und Weise in den Halter 2 eingeführt und fixiert werden. Es müssen also wiederum die drei Klemmelemente 29 bis 31 durch Beaufschlagen einer Kraft in die Einführposition gebracht werden. Dann wird die Flasche 3 mit der Kappe 20 voraus in den Halter 2 eingeführt und auf die Anschlagfläche 26 gedrückt, wodurch der Einstichhohldorn 27 durch die durchstechbare Schicht 21 gestochen wird. In diesem Zustand wird die Kraftbeaufschlagung auf die drei Klemmelemente 29 bis 31 beendet, so dass aufgrund der Federkraft der Federarme 37 und 38 die drei Klemmelemente 29 bis 31 in ihre Klemmposition zurückbewegt werden. Wie in Fig. 12 gezeigt ist, liegt das erste Klemmelement 29 seitlich an der Kappe 20 an. Das zweite Klemmelement 30 liegt am Hals 19 im Bereich der Hinterschneidung 22 an und das dritte Klemmelement 31 liegt ebenfalls am Hals 19 an. Somit bewirken das zweite und dritte Klemmelement 30 und 31 eine Kraftbeaufschlagung in Richtung zur Aufnahmefläche 26, was wiederum ein sicheres Fixieren der Flasche 3 im Halter 2 bewirkt.

Ferner kann am erfindungsgemäßen Flaschenhalter 2 auch eine weitere (nicht gezeigte) Medikamentenflasche 3 fixiert werden, die ein Volumen von z.B. 250 ml aufweist. Bei einer solchen Medikamentenflasche kann der Außendurchmesser der Kappe 20 33 mm und die Höhe der Kappe 14 mm betragen. Im eingesetzten Zustand liegen dann das erste und zweite Klemmelement 29 und 30 an der Kappe 20 an und das dritte Klemmelement 31 greift in die Hinterschneidung 22 ein.

Nachdem der erfindungsgemäße Halter 2 bei der hier beschriebenen Ausführungsform für Kappenhöhen von 7, 10,5 oder 14 mm ausgelegt ist, ist die Dicke der einzelnen Klemmelemente 29-31 bevorzugt 3,5 mm. Sie kann im Bereich von z.B. 3 bis 10 mm, 3 bis 5 mm oder 3,5 bis 4,5 mm liegen.

Die Krümmungsradien der Kreisbahnen 47 und 48 können bevorzugt an die Abmessungen der aufzunehmenden unterschiedlichen Medikamentenflaschen 3 und somit an einen Satz von Flaschen 3 angepasst sein. Der Krümmungsradius des ersten Kreisbahnabschnittes 47 ist bevorzugt größer als der entsprechende Radius der Kappe 20 mit größtem Außendurchmesser des Flaschensatzes. Der Krümmungsradius des zweiten Kreisbahnabschnittes 48 ist bevorzugt kleiner als der entsprechende Radius des Flaschenhalses mit geringstem Außendurchmesser des Flaschensatzes. Damit wird sichergestellt, dass jede der Medikamentenflaschen des Flaschensatzes durch die Durchgangsöffnungen 33, wenn die Klemmelemente in der Einführposition stehen, hindurchbewegt werden können. Die Wahl des Radius des zweiten Kreisbahnabschnittes 48 sorgt dafür, dass stets Teile der beiden linearen Abschnitte 49 und 50 auf die Rückseite der Kappe 20 bzw. in die Hinterschneidung 22 eingreifen, so dass auf die Kappe 20 auf zwei Anlagepunkte oder Anlagebereiche eine Kraft in Richtung zur Anschlagfläche 26 hin ausgeübt wird.

Bei dem hier beschriebenen Ausführungsbeispiel sind drei aufeinandergestapelte Klemmelemente 29-31 vorgesehen. Natürlich kann der erfindungsgemäße Halter auch nur ein oder zwei Klemmelemente oder vier, fünf oder mehr Klemmelemente aufweisen. Des Weiteren ist es möglich, dass die Klemmelemente unterschiedliche Dicken aufweisen. Die Dicken der Klemmelemente können an die Unterschiede der Höhen der verschiedenen aufzunehmenden Flaschen des Flaschensatzes, für den der Flaschenhalter 2 ausgelegt ist, angepasst sein.

Das Halteteil 15 kann insbesondere aus Kunststoff hergestellt sein. Der Kunststoff kann transparent sein. Wie insbesondere in Fig. 8 und 9 ersichtlich ist, kann das Halteteil 15 zwei gegenüberliegende Öffnungen 65, 66 aufweisen, die sich vom Ende 16 hin zum verjüngten Ende 23 des Halteteils 15 erstrecken. Die Länge der Erstreckung kann im Bereich von 0,5 bis 0,25 der Gesamtlänge (bzw. Höhe) des Halteteils 15 betragen. Die Öffnungen 65 und 66 dienen insbesondere dazu, eine Medikamentenflasche 3, die relativ kurz ist, leichter aus dem Halteteil 15 entnehmen zu können.

Die Klemmelemente 29-31 sind bevorzugt aus Kunststoff hergestellt.

Mit dem erfindungsgemäßen Flaschenhalter 2, der auch als Universalkorb bezeichnet werden kann, können somit Flaschen 3 unterschiedlicher Abmessungen problemlos gehalten und fixiert werden.

## Patentansprüche

1. Flaschenhalter zum Halten einer Flasche (3) für eine Spritze,
wobei der Flaschenhalter (2) eine Anschlagfläche (26),
einen über die Anschlagfläche (26) vorstehenden und sich in eine erste Richtung erstreckenden Einstichhohldorn (27),
eine Führung (28) und
ein erstes und ein zweites Klemmelement (29, 30, 31) aufweist,
**dadurch gekennzeichnet, dass**
das erste und das zweite Klemmelement (29, 30, 31) entlang der ersten Richtung aufeinander angeordnet sind und in der Führung (28) quer zur ersten Richtung entlang einer zweiten Richtung bewegbar gelagert sind,
wobei jedes Klemmelement (29-31) eine Durchgangsöffnung (33) umfasst und zwischen einer Einführposition und einer Klemmposition hin und her bewegbar ist,
wobei, wenn die Klemmelemente (29-31) in der Einführposition stehen, ein Hals (19) der Flasche (3) durch die Durchgangsöffnungen (33) bewegt werden kann, bis das vordere Ende der Flasche (3) an der Anschlagfläche (26) anliegt, so dass der Einstichhohldorn (27) eine Schicht (21) an dem vorderen Ende der Flasche (3) durchsticht, und
wobei mindestens eines der Klemmelemente (29-31), wenn es in seiner Klemmposition steht, gegen den Hals (19) der Flasche (3), die mit ihrem vorderen Ende an der Anschlagfläche (26) anliegt, drückt, um dadurch die Flasche (3) im Halter (2) einzuklemmen.

2. Flaschenhalter nach Anspruch 1, bei dem mindestens eines der Klemmelemente (29-31), wenn es in seiner Klemmposition steht, an einer Hinterschneidung (22) am Hals (19) der Flasche (3) anliegt.

3. Flaschenhalter nach Anspruch 1 oder 2, bei dem
jedes Klemmelement (29-31) mindestens einen Federarm (37, 38) aufweist, der das in der Führung (28) gelagerte Klemmelement (29-31) mit einer Kraft in Richtung zur Klemmposition hin beaufschlagt.

4. Flaschenhalter nach Anspruch 3, bei dem jedes Klemmelement (29-31) zwei Federarme (37, 38) aufweist, die V-förmig angeordnet sind.

5. Flaschenhalter nach Anspruch 3 oder 4, bei dem mindestens ein Federarm (37, 38) jedes Klemmelements (29-31) einen ersten und einen zweiten Anschlagsteg (41, 42; 44, 45), die sich von der Durchgangsöffnung (33) weg erstrecken, aufweist,
wobei die beiden Anschlagstege (41, 42; 44, 45) Endpositionen der Bewegung des Klemmelements (29-31) in der zweiten Richtung festlegen.

6. Flaschenhalter nach einem der obigen Ansprüche, bei dem zumindest ein Klemmelement (29-31) einstückig ausgebildet ist.

7. Flaschenhalter nach einem der obigen Ansprüche mit einem hüllenförmigen Halteteil (15) mit einer Wandung (53), wobei die Führung (28) zwei gegenüberliegende Führungsöffnungen (55, 56) in der Wandung (53) umfasst.

8. Flaschenhalter nach einem der obigen Ansprüche, bei dem der Rand der Durchgangsöffnung (33) eines der Klemmelemente (29-31), in der ersten Richtung auf das Klemmelement (29-31) gesehen, zwei entlang der zweiten Richtung gegenüberliegende abgerundete Enden (47, 48) unterschiedlicher Größe umfasst, die mit zwei Verbindungsabschnitten (49, 50) verbunden sind.

9. Flaschenhalter nach Anspruch 8, bei dem
mindestens eines der abgerundeten Enden (47, 48) als Kreisbahnabschnitt ausgebildet ist.

10. Flaschenhalter nach einem der obigen Ansprüche, bei dem
mindestens eines der Klemmelemente (29-31) einen ringförmigen Klemmabschnitt (32) mit der Durchgangsöffnung (33) umfasst.

11. Flaschenhalter nach einem der obigen Ansprüche, bei dem
mindestens eines der Klemmelemente (29-31) um eine Achse entlang der zweiten Richtung um 180° drehsymmetrisch ist.

12. Flaschenhalter nach einem der obigen Ansprüche, bei dem
mindestens eines der Klemmelemente (29-31) entlang der ersten Richtung eine konstante Dicke aufweist.

13. Flaschenhalter nach einem der obigen Ansprüche, bei dem
die Klemmelemente (29-31) unmittelbar aufeinander liegen.

14. Flaschenhalter nach einem der obigen Ansprüche, bei dem alle Klemmelemente (29-31) gleich ausgebildet sind.

15. Spritze mit einem Flaschenhalter (2) nach einem der obigen Ansprüche.

## Claims

1. Bottle holder for holding a bottle (3) for a syringe,
wherein the bottle holder (2) comprises a stop surface (26),
a piercing hollow spike (27) protruding beyond the stop surface (26) and extending in a first direction,
a guideway (28) and
a first and a second clamping element (29, 30, 31),
**characterized in that**
the first and the second clamping element (29, 30, 31) are arranged one on another along the first direction and are mounted in the guideway (28) so as to be moveable along a second direction transverse to the first direction,
wherein each clamping element (29-31) comprises a through-hole (33) and is moveable back and forth between an insertion position and a clamping position, wherein, when the clamping elements (29-31) are in the insertion position, a neck (19) of the bottle (3) can be moved through the through-holes (33) until the front end of the bottle (3) abuts against the stop surface (26) with the result that the piercing hollow spike (27) pierces a film (21) at the front end of the bottle (3), and
wherein at least one of the clamping elements (29-31), when it is in its clamping position, presses against the neck (19) of the bottle (3), which abuts with its front end against the stop surface (26), in order thereby to clamp the bottle (3) in the holder (2).

2. Bottle holder according to claim 1, in which at least one of the clamping elements (29-31), when it is in its clamping position, abuts against an undercut (22) on the neck (19) of the bottle (3).

3. Bottle holder according to claim 1 or 2, in which
each clamping element (29-31) comprises at least one spring arm (37, 38) which applies a force in the direction towards the clamping position to the clamping element (29-31) mounted in the guideway (28).

4. Bottle holder according to claim 3, in which each clamping element (29-31) comprises two spring arms (37, 38) which are arranged in a V shape.

5. Bottle holder according to claim 3 or 4, in which at least one spring arm (37, 38) of each clamping element (29-31) comprises a first and a second stop bar (41, 42; 44, 45), which extend away from the through-hole (33),
wherein the two stop bars (41, 42; 44, 45) define end positions of the movement of the clamping element (29-31) in the second direction.

6. Bottle holder according to one of the above claims, in which at least one clamping element (29-31) is formed in one piece.

7. Bottle holder according to one of the above claims with a shell-shaped holding portion (15) with a wall (53), wherein the guideway (28) comprises two guideway openings (55, 56) lying opposite each other in the wall (53).

8. Bottle holder according to one of the above claims, in which the rim of the through-hole (33) of one of the clamping elements (29-31), seen in the first direction onto the clamping element (29-31), comprises two rounded ends (47, 48) of different sizes lying opposite each other along the second direction, which are connected by two connecting sections (49, 50).

9. Bottle holder according to claim 8, in which
at least one of the rounded ends (47, 48) is formed as a circular path section.

10. Bottle holder according to one of the above claims, in which
at least one of the clamping elements (29-31) comprises an annular clamping section (32) with the through-hole (33).

11. Bottle holder according to one of the above claims, in which
at least one of the clamping elements (29-31) is rotationally symmetrical through 180° about an axis along the second direction.

12. Bottle holder according to one of the above claims, in which
at least one of the clamping elements (29-31) has a constant thickness along the first direction.

13. Bottle holder according to one of the above claims, in which
the clamping elements (29-31) lie directly one on another.

14. Bottle holder according to one of the above claims, in which
all clamping elements (29-31) are formed identical.

15. Syringe with a bottle holder (2) according to one of the above claims.

## Revendications

1. Support de flacon permettant de maintenir un flacon (3) pour une seringue, dans lequel le support de flacon (2) présente
une surface de butée (26),
un perforateur creux (27) faisant saillie à partir de la surface de butée (26) et s'étendant dans une première direction,
un dispositif de guidage (28), et
un premier et un deuxième élément de serrage (29, 30, 31),
**caractérisé en ce que** le premier et le deuxième élément de serrage (29, 30, 31) sont disposés l'un sur l'autre le long de la première direction et sont montés dans le dispositif de guidage (28) mobiles transversalement à la première direction le long d'une deuxième direction,
dans lequel chaque élément de serrage (29 à 31) comprend une ouverture de passage (33) et est mobile alternativement entre une position d'introduction et une position de serrage,
dans lequel, lorsque les éléments de serrage (29 à 31) se trouvent dans la position d'introduction, un goulot (19) du flacon (3) peut être déplacé à travers les ouvertures de passage (33) jusqu'à ce que l'extrémité avant du flacon (3) soit adjacente à la surface de butée (26) de sorte que le perforateur creux (27) perce une couche (21) à l'extrémité avant du flacon (3), et
dans lequel, lorsqu'il se trouve dans sa position de serrage, au moins l'un des éléments de serrage (29 à 31) appuie sur le goulot (19) du flacon (3), dont l'extrémité avant est adjacente à la surface de butée (26), afin de serrer ainsi le flacon (3) dans le support (2).

2. Support de flacon selon la revendication 1, dans lequel, lorsqu'il se trouve dans sa position de serrage, au moins l'un des éléments de serrage (29 à 31) est adjacent à une contre-dépouille (22) sur le goulot (19) du flacon (3).

3. Support de flacon selon la revendication 1 ou 2, dans lequel chaque élément de serrage (29 à 31) présente au moins un bras ressort (37, 38) qui exerce sur l'élément de serrage (29 à 31) monté dans le dispositif de guidage (28) une force en direction de la position de serrage.

4. Support de flacon selon la revendication 3, dans lequel chaque élément de serrage (29 à 31) présente deux bras ressorts (37, 38) qui sont disposés en forme de V.

5. Support de flacon selon la revendication 3 ou 4, dans lequel au moins un bras ressort (37, 38) de chaque élément de serrage (29 à 31) présente une première et une deuxième languette de butée (41, 42 ; 44, 45) qui s'écartent de l'ouverture de passage (33), dans lequel les deux languettes de butée (41, 42 ; 44, 45) définissent des positions d'extrémité du mouvement de l'élément de serrage (29 à 31) dans la deuxième direction.

6. Support de flacon selon l'une quelconque des revendications précédentes, dans lequel au moins un élément de serrage (29 à 31) est réalisé d'un seul tenant.

7. Support de flacon selon l'une quelconque des revendications précédentes, comprenant une partie de retenue sous forme d'enveloppe (15) pourvue d'une paroi (53), dans lequel le dispositif de guidage (28) comprend deux ouvertures de guidage opposées (55, 56) dans la paroi (53).

8. Support de flacon selon l'une quelconque des revendications précédentes, dans lequel le bord de l'ouverture de passage (33) d'un des éléments de serrage (29 à 31), vu sur l'élément de serrage (29 à 31) dans la première direction, comprend deux extrémités arrondies (47, 48), opposées le long de la deuxième direction, de taille différente et qui sont reliées à deux sections de liaison (49, 50).

9. Support de flacon selon la revendication 8, dans lequel au moins l'une des extrémités arrondies (47, 48) est réalisée sous forme de section de trajectoire circulaire.

10. Support de flacon selon l'une quelconque des revendications précédentes, dans lequel au moins l'un des éléments de serrage (29 à 31) comprend une section de serrage annulaire (32) pourvue de l'ouverture de passage (33).

11. Support de flacon selon l'une quelconque des revendications précédentes, dans lequel au moins l'un des éléments de serrage (29 à 31) présente une symétrie de révolution de 180° autour d'un axe le long de la deuxième direction.

12. Support de flacon selon l'une quelconque des revendications précédentes, dans lequel au moins l'un des éléments de serrage (29 à 31) présente une épaisseur constante le long de la première direction.

13. Support de flacon selon l'une quelconque des revendications précédentes, dans lequel les éléments de serrage (29 à 31) sont placés directement les uns sur les autres.

14. Support de flacon selon l'une quelconque des revendications précédentes, dans lequel tous les éléments de serrage (29 à 31) sont réalisés de manière identique.

15. Seringue comprenant un support de flacon (2) selon l'une quelconque des revendications précédentes.
